Europäisches Patentamt

(19) **European Patent Office**

Office européen des brevets

(11) Publication number: **0 242 998 B1**

(12) **EUROPEAN PATENT SPECIFICATION**

(45) Date of publication of patent specification: **03.07.91**

(51) Int. Cl.5: **A61L 2/18**, A01N 59/02, A01N 37/10, //(A01N59/02, 37:10)

(21) Application number: **87302491.3**

(22) Date of filing: **23.03.87**

Divisional application 90201672.4 filed on 23/03/87.

(54) Disinfecting composition.

(30) Priority: **22.03.86 GB 8607160**
**30.04.86 GB 8610521**
**08.05.86 GB 8611190**

(43) Date of publication of application:
**28.10.87 Bulletin 87/44**

(45) Publication of the grant of the patent:
**03.07.91 Bulletin 91/27**

(84) Designated Contracting States:
**AT BE CH DE ES FR GB IT LI NL SE**

(56) References cited:
**EP-A- 0 139 994      WO-A-85/01209**
**WO-A-86/05401      AU-B- 528 853**
**GB-A- 1 601 430      US-A- 3 910 296**

(73) Proprietor: **SMITH & NEPHEW plc**
**2 Temple Place Victoria Embankment**
**London WC2R 3BP(GB)**

(72) Inventor: **Drain, David John**
**302 Knightsfield**
**Welwyn Garden City, Hertfordshire(GB)**
Inventor: **Howes, John Gordon Bernard**
**'Larond', Heath Road,**
**Hertford Heath, Hertfordshire,(GB)**
Inventor: **Hollingsbee, Derek Andrew**
**4 Fanners Green Cottages**
**Great Waltham, Chelmsford, Essex(GB)**

(74) Representative: **Cole, William Gwyn**
**Corporate Patents Department Smith and**
**Nephew Research Limited Gilston Park**
**Harlow Essex CM20 2RQ(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services

## Description

The present invention relates to disinfection of contact lenses and more particularly to disinfection processes which involve contacting the lens to be disinfected with an aqueous solution containing sulphur dioxide and more particularly to the use of an aqueous solution containing sulphur dioxide generated from a sulphur dioxide-liberating compound and benzoic acid; to compositions used in such disinfection processes and to packs to enable the processes to be carried out.

Contact lenses when worn daily can be come contaminated with dirt, cosmetics, tear debris, proteins, microorganisms and the like which if they are not removed from the lens can cause irritation when the lens is replaced in the eye. It is advisable, therefore, that contact lenses, and soft contact lensES in particular, are cleaned and disinfected on a regular basis, preferably daily.

It has long been recognised that the disinfection of contact lenses and soft contact lens in particular, is beset with problems. Chemical methods have been considered. These methods reduce the propensity to cause a protein build-up on the lens caused by heat disinfection methods but may instead suffer from the disadvantage that the chemical agent may concentrate in the lens. When the lens is replaced in the eye, the chemical agent may be released from the lens and damage the cornea. Certain chemical disinfectants, for example theose containing halogen or halogen oxyacids, have been found to damage certain lens polymers. It would be advantageous therefore to have a cold disinfecting process for contact lenses which was especially suitable for use with soft contact lens, in which the disinfecting means did not cause protein deposition, did not concentrate in the lens, did not affect lens polymers and could be made safe to allow direct replacment of the lens into the eye after the disinfecting process. It has been found that these criteria are met if the disinfecting process employs as a disinfecting medium an aqueous solution of sulphur dioxide and preferably of a synergistic mixture of sulphur dioxide and benzoic acid.

This process is suitable for disinfecting hard, hydrophilic (soft) and gas permeable contact lenses but is particularly suitable for disinfecting soft contact lenses.

Accordingly the present invention provides an aqueous solution suitable for the disinfection of a contact lens containing sulphur dioxide and benzoic acid in disinfecting amounts.

The present invention also provides a method for disinfecting a contact lens wherein the lens is contacted for a period of time sufficient to disinfect the lens with an aqueous solution containing a sulphur dioxide and benzoic acid.

Compounds which will liberate sulphur dioxide in the presence of water are well known. Metabisulphites and dithionites, for example, will decompose in the presence of water with the liberation of sulphur dioxide and these compounds have been found to be suitable for use as sulphur dioxide liberating compounds for use in the process of the invention. They are conventionally used in the form of their ammonium or alkali metal salts of which sodium salts are preferred and particularly favoured is the use of sodium metabisulphite. This compound has been used as an antioxidant in adrenaline eye drops and is known to be non-irritant when instilled in the eye, however its use as a disinfecting agent in ophthalmic applications has not been disclosed or suggested.

The amount of sulphur dioxide-liberating compound may be such as to provide a concentration of sulphur dioxide in solution of aptly 40 to 5000 ppm, and suitably 50 to 500 ppm, more suitably 75 to 400 ppm and preferably 100 to 250 ppm of sulphur dioxide.

The disinfecting solution may therefore be presented either in a preformed solution or as a solid form for dissolving in water or in a solution of tonicity agent for example sodium chloride and/or buffering agent and the like. Suitable water may include distilled water, deionised water or tap water. Since sulphur dioxide-liberating compounds do so under acidic conditions the tablet or water it is dissolved in should contain an acidic component to provide the initial solution with a pH of from 1.8 to 5.5, more suitably 1.8 to 4.0 and preferably 1.8 to 3.2 for example 2.0 and 3.0.

Suitable acidifying agents may include sorbic benzoic, tartaric, ascorbic, lactic, acetic, fumaric, citric, maleic, adipic and malic acid, or inorganic mineral acids such as hydrochloride or phosphoric acid.

Surprisingly it has been found that sulphur dioxide and benzoic acid exhibit synergistic antibacterial activity against several important organisms and especially Candida albicans when they are employed in an aqueous solution at a pH of from 1.8 to 3.2.

In another aspect the method of the present invention provides a method for disinfecting a contact lens wherein the lens is contacted for a period of time sufficient to disinfect the lens with a solution containing an effective amount of a synergistic mixture of sulphur dioxide and benzoic acid and then adjusting the pH of the solution so that when the disinfected lens is removed from the solution and replaced in the eye it is non-irritant to the eye. Suitably the pH of the solution will be adjusted to the range of 6 to 8.

Suitably the compositions used in this aspect of the present invention will contain from 0.01 to 0.25%

2

by weight of benzoic acid, more suitably 0.05 to 0.225% and preferably from 0.1 to 0.2%.

(When used herein the term % refers to % by weight).

The amount of sulphur dioxide-liberating compound which is to be used with benzoic acid may suitably be such as to provide a concentration of sulphur dioxide in solution of aptly 10 to 5000 ppm and suitably 10 to 500 ppm, more suitably 15 to 200 ppm and preferably 20 to 100 ppm of sulphur dioxide for example 25 ppm.

The skilled worker will appreciate that an effective disinfecting amount of sulphur dioxide and benzoic acid in aqueous solution will vary according to the pH value of the solution which contains them. However, the compositions used in the present invention will generally contain the amounts given above.

The final composition of the disinfectant solution may be selected so as to give a solution which is substantially isotonic that is it will have a tonicity equivalent to a 0.7 to 1.2% solution of sodium chloride. The solutions containing an effective disinfecting amount of benzoic acid and/or sulphur dioxide-liberating compound are normally hypotonic and thus the tonicity of such solutions should be adjusted by the components of the neutralising system as described herein after and/or by a tonicity agent such as sodium chloride.

The period of time envisaged for disinfection of a contact lens is from 10 minutes to 8 hours. If desired the system can be used for disinfecting a lens by allowing it to stand overnight in contact with the disinfecting solution.

Since replacement of a lens into the eye from a solution of low pH value for example 1.8 to 3.2 would be irritant, it is necessay to adjust the pH of the solution containing it for example to the range 6 to 8 before the lens can be removed and replaced safely directly into the eye. This adjustment may be achieved by adding agent such as sterile alkali solution or tablet containing material which has an alkaline reaction with water to the solution containing the lens at the end of the disinfection cycle. There is a risk that this step may be omitted and a lens from a non-neutralised solution may be replaced in the eye. It is preferred instead to add at the beginning of the disinfection process a neutralising system which will slowly release or provide delayed release of agent which will adjust the pH of the solution so that wehn the disinfected lens is removed from the disinfecting solution, the solution has a pH of from 6 to 8. The adjustment of the pH OF the solution may be facilitated by manually mixing the contents from time to time and especially just prior to removing the lens.

Suitably the aqueous solutions may additionally contain an acidic component as hereinbefore described. Preferably the acidic component is tartaric acid or citric acid.

The aqueous solution will aptly contain the proportions of sulphur dioxide and benzoic acid as hereinbefore described.

Thus in a preferred aspect the invention provides an aqueous solution suitable for the disinfection of a contact lens whioh solution contains from 0.1 to 0.25% benzoic acid and from 10 to 500 ppm of sulphur dioxide and which solution has a pH of from 1.8 to 3.2.

Conventionally a contact lens is disinfected by contacting it in a container, which may be closed by a screw cap, with an aqueous solution containing the disinfecting agent. The disinfecting agent may therefore be present either in a preformed solution or as a solid form for dissolving in water. Suitable water may include distilled water, deionised water or tap water. Since benzoic acid and sulphur dioxide are antimicrobial only under acidic conditions the tablet or water they are dissolved in may, contain an additional acidic component to provide the initial solution with a pH of from 1.8 to 3.2, and preferably provide a pH of 2.0 to 3.0 for example 2.0, 2.5 or 3.0

Thus the presentation of the system with which to carry out the method of the invention may include

(a) an aqueous solution into which is added a tablet of the benzoic acid and sulphur dioxide liberating compound and a tablet adapted to slowly release or delayed release a material having an alkaline reaction with water.

(b) a two layer tablet which has a rapidly dissolving layer which contains benzoic acid and sulphur dioxide-liberating compound and optionally an acidifying agent and a slowly dissolving or delayed release layer containing the neutralising agent. Such a tablet may be added to water in which the lens is immersed or if the acidifying agent is not present in the tablet, the tablet may be added to acidified water.

(c) an aqueous solution containing benzoic acid and an acidic component, a tablet which contains a sulphur dioxide-liberating compound and a slowly dissolving or delayed release tablet containing a material which has an alkaline reaction with water.

Aptly the material which has an alkaline reaction with water may be added as a tablet and preferably one which has been adapted to provide slow or delayed release of the material, for example by encapsulation either as particles of as a tablet in a water soluble or water permeable polymer coating, in a

3

solid matrix or in a hard slowly dissolving tablet. Suitable materials include sodium borate, carbonates and bicarbonates such as sodium carbonate or bicarbonate and disodium hydrogen phosphate or other salts which give an alkaline reaction or dissolution in water.

Preferably the alkaline material is present in a tablet which is encapsulated in a water soluble polymer coating. Apt coatings may be formed from materials which include water soluble cellulosic materials such as hydroxypropyl, cellulose, hydroxypropyl methyl cellulose, water soluble polymers such as polyvinyl alcohol, polyacrylic acids such as those known as Carbopols (trade mark) Eudragits (trade mark). The coatings may additionally contain a more water soluble material such as lactose to facilitate the solubilisation of the coating. In a particularly preferred form the encapsulated tablet may be effervescent on exposure to water. This has the effect that as the coating dissolves from the tablet it is exposed to water it begins to effervesce thereby mixing the contents of the container and disrupting any remaining coating so providing rapid release of the alkaline material. The skilled worker will appreciate that the thickness of the coating and its solubility will dictate the time of the delayed release of the alkaline material. Aptly the effervescence may be provided by an alkaline compound if this compound is for example a carbonate or bicarbonate such as sodium or potassium carbonate or bicarbonate. Aptly an acidic compound is also present in the tablet also to help generate the effervescent gas. Suitable acidic agents include citric, tartaric or malic acids. Naturally the alkaline material will be in excess so that the pH of the solution is adjusted to pH 6 to 8.

The delayed release coating to the tablet may be applied by many processes including the following, placing the tablet within a water soluble or water permeable envelope; coating the tablet by spraying or fluidised bed; dip coating by immersing the tablet in a solution of the polymer and drying in warm air, suitable polymer solutions include hydroxypropyl cellulose in ethyl alcohol, hydroxypropyl methyl cellulose in aqueous alcohol; compression coating a tablet centred in a dry polymer·powder. Apt processes are dip coating and compression coating. Typically a $\frac{1}{4}$ " tablet containing the neutralising agent and/or tonicity adjusting agent may be coated by compressing in a $\frac{3}{8}$ " tablet press surrounded by a dry polymer powder. The coating so formed is about $^1/_{16}$ " thick and may dissolve in 2-3 hours releasing the contents of the tablet the contents of which then dissolve.

In a further aspect the present invention comprises a three part pack for producing a contact lens disinfecting solution, said three part pack comprising one part an aqueous solution of an effective disinfecting amount of benzoic acid, a second part containing a predetermined quantity of a sulphur dioxide liberating compound and another part comprising a predetermined quantity of a tablet adapted to slowly release or delay the release of a material having an alkaline reaction with water.

In a further aspect the present invention comprises a two part pack for producing a contact lens disinfecting solution, said two part pack comprising a predetermined quantity of a tablet containing an effective disinfecting amount of benzoic acid and/or sulphur dioxide-liberating compound and a slowly dissolving or delayed release neutralising agent and another part comprising a predetermined quantity of an aqueous vehicle.

The disinfecting system or the neutralising system or both may also contain a metal ion chelating agent. The chelating agent may be any compound which will chelate at least calcium and magnesium ions from tap water and which is compatible with the other components and with ophthalmic use, that is the chelating agent must be non-irritant to the eye at the concentrations used in the solution. Suitable chelating agents will include ethylene diamine tetra-acetic acid and salts thereof for example the disodium salt and complex polyphosphate for example sodium hexametaphosphate, sodium pyrophosphate or sodium tripolyphosphate.

The chelating agent is used in an amount sufficient to remove calcium and magnesium ions from the tap water used to form the disinfecting solution. The skilled worker will appreciate that the minimum amount required will vary depending upon the hardness of the tap water, a minimum amount of at least 0.01% by weight will be generally used. Suitably the amount of chelating agent used will from 0.01 to 1% by weight, more suitably 0.02 to 0.50% and preferably 0.05 to 0.1%.

The use of the term tap water refers to water which has not be de-ionised or specially purified but is sufficiently uncontaminated to be of a potable standard. Conventionally such water is supplied as drinking water from treatment plants to a tap. This term also includes water of equivalent quality from other sources such as spring water.

Tap water may also have been chlorinated and therefore the disinfecting system may also include a dechlorinating agent. The disinfecting compositions of the present invention may use a dechlorinating agent a metabisulphite especially sodium metabisulphite.

An indicator can be included in the system so that the user may visually determine that the pH value of the disinfecting solution has been adjusted to the range 6 to 8.

The compositions employed in the method of the invention may also contain a surface active agent to assist in the cleaning and/or wetting of the lenses by the disinfecting composition. This is particularly useful when the lenses being treated are hard or gas permeable lenses. Suitable surface active agents such as polyoxyethylene-polyoxypropylene diol block copolymers, sorbitan fatty acid esters, polyoxyethylated sorbitan fatty acid esters and the like. Suitably the composition may contain from 0.01 to 1% of non-ionic surface active agent and preferable 0.05 to 0.5%.

Example 1

A solution of benzoic acid (20mg) and sodium metabisuphite (7.2mg) in distilled water (10ml) is sterilised by filtration through a 0.22µm polyester filter. The final solution has a pH of 3.0 (approx). A lens is immersed in this solution in a closed container for 4 hours. At the end of this period the contents of a sachet containing anhydrous disodium hydrogen phosphate (160mg) and potassium dihydrogen phosphate (15mg), which are previously sterilised by gamma irradiation, are added to the solution containing the lens and the container shaken to dissolve the powder. The resultant solution has a pH value of 7.0 and is approximately isotonic the lens now disinfected is removed from the solution and is replaced in the eye.

Example 2

A sterile solution of benzoic acid and sodium metabisulphite in distilled water is prepared as described in Example 1. A lens is immersed in the solution in a closed container for 6 hours. At the end of this period a compressed tablet containing anhydrous disodium hydrogen phosphate (160mg) and potassium dihydrogen phosphate (15mg) is added to the closed·container and the container and contents shaken to dissolve the tablet. The final solution has a pH of 7.4 and is approximately isotonic. The disinfected lens may be removed from the solution and replaced in the eye.

Example 3

A sterile solution (7ml) of benzoic acid and sodium metabisulphite (225mg and 60mg in distilled water) prepared as described in Example 1 are taken and placed in a closable container. A lens is immersed in the solution in the container for 4 hours.

At the end of this period 3ml of sterile solution of disodium hydrogen phosphate and potassium dihydrogen phosphate (8.0mg and 0.75g respectively dissolved in 100ml of distilled water and sterilised by filtration) are added to the container. The resultant solution is approximately isotonic and has a pH of 7.2. The disinfected lens may be removed from the solution and replaced in the eye.

Example 4

An aqueous solution of benzoic acid and sodium metabisulphite (10mls containing 20mg and 7.2mg respectively) is placed in a screw-topped container. A tablet comprising:

| | |
|---|---|
| Sodium bicarbonate | 95mg |
| Tartaric acid | 25mg |
| Disodium EDTA 2H$_2$O | 10mg |
| Disodium hydrogen phosphate | 10mg |
| Sodium chloride | 15mg |
| Polyethylene glycol | 5mg |

coated with hydroxy propyl cellulose is then added to the container. The tablet is made by compressing together the above ingredients to form a tablet approximately ¼ " in diameter. A coat of hydroxy propyl cellulose is then compressed around it using a ⅜ " tablet punch to give a coated thickness of about ¹/₁₆ " The lens is then immersed in the solution in the container. After about 2.5 hours the coating has dissolved sufficiently for the tablet to begin to effervesce. The tablet dissolves into solution so that the final solution is substantially isotonic and has a pH of 7.

Test solution containing 62.5ppm sulphur dioxide acidified at pH = 2.0 with citric acid.

| Test Organisms | Viable count $\log_{10}$ count/ml Sampled after the following hours | | |
|---|---|---|---|
| | 0 hours | 3 hours | 24 hours |
| Staph aureus | 5.61 | <1.0 | <1.0 |
| Pseudomonas aeruginosa | 4.80 | <1.0 | <1.0 |
| E. Coli | 5.38 | <1.0 | <1.0 |
| C. albicans | 5.22 | 4.55 | <1.0 |
| A. niger | 5.6 | <1.0 | <1.0 |
| S. marcescens | 5.83 | <1.0 | <1.0 |

A suspension of Candida albicans (ATCC 10231) at concentration of $10^8$ organisms/ml was prepared. Aliquots (20ml) of the test solutions containing benzoic acid, sulphur dioxide and a mixture of benzoic acid and sulphur dioxide were innoculated with $20\mu l$ of the suspension of the test organism and mixed. The solutions were then sampled immediately and at 1 hours and 3 hours after mixing by removing 1ml of solution and placing it into 9mls of a neutralising solution (Tryptone soya broth containing Tween (Trade Mark) and (lecithin). The number of organisms retrievable from the solution was then determined.

| Solution | pH | Viable count $\log_{10}$ count/ml Sampled after the following hours | | |
|---|---|---|---|---|
| | | 0 | 1 | 3 |
| 0.2% Benzoic acid | 2.0 | 6.03 | 5.19 | 2.78 |
| 0.2% Benzoic acid | 3.0 | 5.84 | 5.65 | 5.01 |
| 25ppm Sulphur dioxide | 2.0 | 5.76 | 5.84 | 5.75 |
| 500ppm Sulphur dioxide | 3.0 | 5.89 | 5.86 | 5.61 |
| 0.2% benzoic acid/ 25ppm sulphur dioxide | 2.0 | 5.93 | 3.77 | <1.0 |
| 0.2% Benzoic acid/ 500ppm Sulphur dioxide | 3.0 | 5.94 | 4.82 | <1.0 |

The results show synergy between sulphur dioxide and benzoic acid against Candida albicans in aqueous solutions which have pH of 2.0 to 3.0.

**Claims**

1. An aqueous solution suitable for the disinfection of a contact lens containing sulphur dioxide and

6

benzoic acid in effective disinfecting amounts.

2. Aqueous solution as claimed in claim 1 having a pH of from 1.8 to 3.2.

3. An aqueous solution as claimed in claim 1 or claim 2 in which the solution contains from 10 to 5000 ppm of sulphur dioxide.

4. An aqueous solution as claimed in any of claims 1 to 3 in which the solution contains from 0.01 to 0.25% benzoic acid.

5. An aqueous solution as claimed in any one of claims 1 to 3 in which the solution contains from 0.01 to 0.25% benzoic acid and from 10 to 500ppm of sulphur dioxide and which has a pH of from 1.8 to 3.2.

6. An aqueous solution as claimed in any one of the preceding claims in which the solution additionally contains an acidic component.

7. An aqueous solution as claimed in claim 6 in which the acidic component is tartaric acid.

8. An aqueous solution as claimed in any one of the preceding claims to which has been added a material which has an alkaline reaction to water to adjust the pH to 6 to 8 whereby the final solution is substantially isotonic.

9. An aqueous solution as claimed in any one of the preceding claims in which the solution contains from 0.1 to 1% of chelating agent.

10. An aqueous solution as claimed in any one the preceding claims in which the solution contains an indicator which allows visual determination that the pH value of the disinfecting solution has been adjusted to the range pH 6 to 8.

11. A three part pack for producing a contact lens disinfecting solution, said three part pack comprising one part an aqueous solution of an effective disinfecting amount of benzoic acid and acidic component, a second part comprising a predetermined quantity of a tablet containing a sulphur dioxide liberating compound and another part comprising a predetermined quantity of a tablet adapted to slowly release or delay the release of a compound having an alkaline reaction with water.

12. A pack as claimed in claim 11 wherein the sulphur dioxide liberating compound is sodium metabisulphite.

13. A method for disinfecting a contact lens wherein the lens is contacted for a period of time sufficient to disinfect the lens with an aqueous solution containing sulphur dioxide and benzoic acid.

14. A method as claimed in claim 13 in which the pH value of the solution is adjusted so that when the disinfected lens is removed from the solution, the solution has a pH value of from 6 to 8.

15. A method as claimed in claim 14 in which the pH of the solution is adjusted by means of the addition to the solution of a material which has an alkaline reaction to water.

16. A method as claimed in claim 15 in which the material which has an alkaline reaction to water is present in the form which is adapted to provide slow or delayed release of the material.

17. A method as claimed in claim 14 or claim 15 in which the pH of the solution is adjusted by adding an effective amount of disodium hydrogen phosphate and potassium dihydrogen phosphate.

18. A method as claimed in any one of claims 14 to 17 in which the final composition of the disinfecting solution is substantially isotonic.

19. A method as claimed in any one of claims 13 to 18 in which there is included in the system an indicator which allows visual determination that the pH of the disinfecting solution has been adjusted to the range of pH 6 to 8.

**Revendications**

1. Solution aqueuse convenable pour la désinfection d'une lentille de contact, caractérisée en ce qu'elle contient du dioxyde de soufre et de l'acide benzoique en quantités efficaces pour la désinfection.

2. Solution aqueuse suivant la revendication 1, caractérisée en ce qu'elle a un pH de 1,8 à 3,2.

3. Solution aqueuse suivant la revendication 1 ou la revendication 2, caractérisée en ce qu'elle contient de 10 à 500 ppm de dioxyde de soufre.

4. Solution aqueuse suivant l'une quelconque des revendications 1 à 3, caractérisée en ce que la solution contient de 0,01 à 0,25 % d'acide benzoique.

5. Solution aqueuse suivant l'une quelconque des revendications 1 à 3, caractérisée en ce que la solution contient de 0,01 à 0,25 % d'acide benzoique et de 10 à 500 ppm de dioxyde de soufre et qu'elle a un pH de 1,8 à 3,2.

6. Solution aqueuse suivant l'une quelconque des revendications précédentes, caractérisée en ce qu'elle contient de plus un composant acide.

7. Solution aqueuse suivant la revendication 6, caractérisée en ce le composant acide est l'acide tartrique.

8. Solution aqueuse suivant l'une quelconque des revendications précédentes, caractérisée en ce qu'elle est additionnée d'une matière qui a une réaction alcaline avec l'eau pour ajuster le pH à une valeur de 6 à 8, de telle sorte que la solution finale est sensiblement isotonique.

9. Solution aqueuse suivant l'une quelconque des revendications précédentes, caractérisée en ce qu'elle contient de 0,1 à 1 % d'agent chélatant.

10. Solution aqueuse suivant l'une quelconque des revendications précédentes, caractérisée en ce qu'elle contient un indicateur qui permet de déterminer à l'oeil si le pH de la solution désinfectante a été ajusté dans la gamme de pH 6 à 8.

11. Conditionnement à trois parties pour produire une solution désinfectante pour lentilles de contact, caractérisé en ce qu'une partie de ce conditionnement comprend une solution aqueuse d'une quantité désinfectante efficace d'acide benzoique et de composant acide, qu'une deuxième partie comprend une quantité prédéterminée d'un comprimé contenant un composé libérant du dioxyde de soufre et qu'une troisième partie comprend une quantité prédéterminée d'un comprimé conçu pour libérer lentement ou retarder la libération d'un composé ayant une réaction alcaline avec l'eau.

12. Conditionnement suivant la revendication 11, caractérisé en ce que le composé libérant le dioxyde de soufre est du métabisulfite de sodium.

13. Procédé pour désinfecter une lentille de contact, caractérisé en ce que la lentille de contact est mise en contact, pendant une période de temps suffisante pour la désinfecter, avec une solution aqueuse contenant du dioxyde de soufre et de l'acide benzoique.

14. Procédé suivant la revendication 13, caractérisé en ce que le pH de la solution est ajusté de telle sorte que lorsque la lentille désinfectée est sortie de la solution, cette solution a un pH de 6 à 8.

15. Procédé suivant la revendication 14, caractérisé en ce que le pH de la solution est ajusté par addition à celle-ci d'une matière qui a une réaction alcaline avec l'eau.

16. Procédé suivant la revendication 15, caractérisé en ce que la matière qui a une réaction alcaline avec l'eau est présente sous la forme qui est conçue pour fournir une libération lente ou retardée de la matière.

EP 0 242 998 B1

17. Procédé suivant la revendication 14 ou la revendication 15, caractérisé en ce que le pH de la solution est ajusté par addition d'une quantité efficace de phosphate acide disodique et de phosphate diacide de potassium.

18. Procédé suivant l'une quelconque des revendications 14 à 17, caractérisé en ce que la composition finale de la solution désinfectante est sensiblement isotonique.

19. Procédé suivant l'une quelconque des revendications 13 à 18, caractérisé en ce que le système comprend un indicateur qui permet de déterminer à l'oeil si le pH de la solution désinfectante a été ajusté dans la gamme de pH 6 à 8.

**Ansprüche**

1. Eine zur Desinfektion von Kontaktlinsen geeignete wässrige Lösung, die Schwefeldioxid und Benzoesäure in zur Desinfektion wirksamen Mengen enthält.

2. Eine wässrige Lösung nach Anspruch 1, dadurch gekennzeichnet, daß die Lösung einen pH von 1,8 bis 3,2 besitzt.

3. Eine wässrige Lösung nach den Ansprüchen 1 oder 2, dadurch gekennzeichnet, daß die Lösung 10 bis 5000 ppm Schwefeldioxid enthält.

4. Eine wässrige Lösung nach den Ansprüchen 1 - 3, dadurch gekennzeichnet, daß die Lösung 0,01 bis 0,25 % Benzoesäure enthält.

5. Eine wässrige Lösung nach den Ansprüchen 1 - 3, dadurch gekennzeichnet, daß die Lösung 0,01 bis 0,25 % Benzoesäure und 10 bis 500 ppm Schwefeldoxid enthält und einen pH von 1,8 bis 3,2 besitzt.

6. Eine wässrige Lösung nach den Ansprüchen 1 - 5, dadurch gekennzeichnet, daß die Lösung zusätzlich eine saure Komponente enthält.

7. Eine wässrige Lösung nach Anspruch 6, dadurch gekennzeichnet, daß die saure Komponente Weinsäure ist.

8. Eine wässrige Lösung nach den Ansprüchen 1 - 7, dadurch gekennzeichnet, daß zu der Lösung zusätzlich ein Material hinzugefügt wurde, das in Wasser eine alkalische Reaktion aufweist, um den pH auf 6 - 8 einzustellen, wobei die fertige Lösung im wesentlichen isotonisch ist.

9. Eine wässrige Lösung nach den Ansprüchen 1 - 8, dadurch gekennzeichnet, daß die Lösung 0,1 bis 1 % eines Chelatzusatzes enthält.

10. Eine wässrige Lösung nach den Ansprüchen 1 - 9, dadurch gekennzeichnet, daß die Lösung einen Indikator enthält, der die visuelle Ermittlung erlaubt, daß der pH der Desinfektionslösung auf den Bereich 6 - 8 eingestellt ist.

11. Eine dreiteilige Packung zur Herstellung einer Desinfektionslösung für Kontaktlinsen, wobei die dreiteilige Packung einen Teil enthält mit einer wässrigen Lösung einer zur Desinfektion wirksamen Menge an Benzoesäure und einer sauren Komponente, einen zweiten Teil mit einer vorher ermittelten Menge einer Tablette, die eine Verbindung enthält, welche Schwefeldioxid freisetzt und einen dritten Teil, der eine vorher ermittelte Menge einer Tablette enthält, die dazu geeignet ist, eine Verbindung abzugeben oder die Abgabe derselben zu verzögern, die mit Wasser eine alkalische Reaktion aufweist.

12. Eine Packung nach Anspruch 11, dadurch gekennzeichnet, daß die Schwefeldioxid freisetzende Verbindung Natrium-meta-bisulfit ist.

13. Ein Verfahren zur Desinfektion einer Kontaktlinse, dadurch gekennzeichnet, daß die Linse für eine ausreichend lange Zeit mit einer wässrigen Lösung in Kontakt gebracht wird, die Schwefeldioxid und Benzoesäure enthält.

9

14. Ein Verfahren nach Anspruch 13, dadruch gekennzeichnet, daß der pH-Wert der Lösung so eingestellt ist, daß die Lösung einen pH-Wert von 6 - 8 hat, wenn die Linse aus der Lösung entfernt wird.

15. Verfahren nach Anspruch 14, dadurch gekennzeichnet, daß der pH der Lösung dadurch eingestellt wird, daß ein Material mit alkalischer Reaktion in Wasser zugesetzt wird.

16. Verfahren nach Anspruch 15, dadurch gekennzeichnet, daß das Material mit alkalischer Reaktion in Wasser in einer solchen Form vorliegt, die dazu geeignet ist, eine langsame oder verzögerte Abgabe des Materials herbeizuführen.

17. Verfahren nach den Ansprüchen 14 oder 15, dadurch gekennzeichnet, daß der pH der Lösung durch Zugabe einer wirksamen Menge von Dinatriumhydrogenphosphat und Kaliumdihydrogenphosphat eingestellt wird.

18. Verfahren nach den Ansprüchen 14 - 17 dadurch gekennzeichnet, daß die fertige Zusammensetzung der Desinfektionslösung im wesentlichen isotonisch ist.

19. Verfahren nach den Ansprüchen 13 - 18, dadurch gekennzeichnet, daß in dem System ein Indikator enthalten ist, der die visuelle Ermittlung erlaubt, daß der pH der Desinfektionslösung auf den Bereich pH 6 - 8 eingestellt ist.